Europäisches Patentamt

European Patent Office

Office européen des brevets

(11) Veröffentlichungsnummer: **0 279 401**
**A2**

## (12) EUROPÄISCHE PATENTANMELDUNG

(21) Anmeldenummer: 88102200.8

(22) Anmeldetag: 15.02.88

(51) Int. Cl.⁴: **A61L 2/18** , G02C 13/00 , C11D 3/48 , C11D 3/386

(30) Priorität: 16.02.87 DE 3704823

(43) Veröffentlichungstag der Anmeldung:
24.08.88 Patentblatt 88/34

(84) Benannte Vertragsstaaten:
AT CH DE ES GR LI NL

(71) Anmelder: Dr. Thilo & Co. GmbH
Rudolf-Diesel-Ring 21
D-8029 Sauerlach(DE)

(72) Erfinder: Tschöpe, Michael, Dr.
Bellinzonastrasse 1
D-8000 München 71(DE)

(74) Vertreter: von Füner, Alexander, Dr. et al
Patentanwälte v. Füner, Ebbinghaus, Finck
Mariahilfplatz 2 & 3 Postfach 95 01 60
D-8000 München 95(DE)

(54) **Kontaktlinsenpflegemittel mit desinfizierender und reinigender Wirkung.**

(57) Die Erfindung betrifft ein Kontaktlinsenpflegemittel mit desinfizierender Wirkung mit einem Gehalt an chlorfreisetzenden Verbindungen, das dadurch gekennzeichnet ist, daß es zur Kombination mit einer reinigenden Wirkung a) eine chlorfreisetzende Verbindung enthält und 0,5 bis 100 ppm verfügbares Chlor in der Gebrauchslösung eufweist, b) eine oder mehrere Proteasen enthält und eine proteolytische Aktivität von 1 bis 1000 E in der Gebrauchslösung aufweist, c) übliche Formulierungshilfsstoffe enthält, sowie ein Verfahren zum Pflegen von Kontaktlinsen durch Behandeln mit chlorfreisetzenden Verbindungen, wobei der Wirkungseintritt der Enzyme gegenüber dem Wirkungseintritt der chlorfreisetzenden Verbindung zeitlich verzögert wird.

EP 0 279 401 A2

# KONTAKLINSENPFLEGEMITTEL MIT DESINFIZIERENDER UND REINIGENDER WIRKUNG

Vorliegende Erfindung betrifft Kontaktlinsenpflegemittel mit desinfizierender und reinigender Wirkung.

Nach Untersuchungen von Herrn Roth, Universitätsklinik Ulm, sind fast die Hälfte (46,3%) der Unverträglichkeitsreaktionen beim Tragen von Kontaktlinsen auf ungenügende Reinigung und mangelhafte Desinfektion der Linsen zurückzuführen. Neben Ruß-und Staubpartikeln aus der Umwelt, Resten von Kosmetika sowie Epithelresten lagern sich an Kontaktlinsen auch Proteine aus der Tränenflüssigkeit, Lipide aus den Meibomschen Drüsen sowie Mucopolysaccharide an. Bei ungenügender Reinigung wird die Kontaktlinse zunehmend schlechter benetzbar. Hinzu kommt, daß die Ablagerungen als Bakteriennährböden dienen können. Die in den Ablagerungen eingebetteten Bakterien sind dann einer Desinfektion unter Umständen nicht mehr zugänglich und können zu Infektionen des Auges führen. Aus diesem Grunde kommt der möglichst vollständigen Reinigung neben der Desinfektion der Kontaktlinsen eine wichtige Rolle zur Erhaltung der Verträglichkeit zu. Neben der regelmäßigen Reinigung ist die Desinfektion der Kontaktlinsen eine weitere wichtige Voraussetzung für beschwerdefreies Tragen von Kontaktlinsen. Durch Verwendungsfehler herkömmlicher Desinfektionssysteme sowie durch ungenügende Mitarbeit des Kontaktlinsenträgers kann es zu erheblichen mikrobiellen Verunreinigungen der Linse und des Aufbewahrungsbehälters kommen. Neben Enterobakterien wurden Pseudomonaden sowie Schimmelpilze in ungenügend gepflegten Kontaktlinsenbehältern sowie auf den Kontaktlinsen selbst gefunden.

Die bislang verwendeten Methoden zur Desinfektion von Kontaktlinsen weisen unter anderem den Nachteil auf, nicht gleichzeitig in ausreichendem Maße zu reinigen. Die früher angewandte Desinfektion von weichen Kontaktlinsen durch Hitze führte, zum Beispiel bei ungenügender Vorreinigung der Linsen, zu einem Festbacken, insbesondere der Proteinablagerungen, wodurch sich äußerst schwer zu entfernende Beläge ergaben. Die zur sogenannten Kaltsterilisierung verwendeten Aufbewahrungslösungen wirken aufgrund ihres Gehaltes an Konservierungsmittel desinfizierend. Neben der Allergisierungsgefahr durch die Verwendeten Konservierungsmittel, z.B. Thiomersal oder Chlorhexidindiglukonat, wurde zudem festgestellt, daß durch die Konservierungsmittel eine Fixierung ungenügend entfernter Ablagerungen erfolgt, so daß diese Lösungen Ablagerungsprobleme auf Kontaktlinsen noch verstärken können (Ehrich W., Zeitschrift für Praktischen Augenheilkunde, 4 -

(1983) 77-78).

Auch Wasserstoffperoxidlösungen von 0,6 bis 3,0%, die zur Desinfektion von Kontaktlinsen eingesetzt werden, weisen nur eine ungenügende Reinigunosaktivität auf (W. Ehrich, Contactologia 7D, 126-129 (1985)). Die früher vertretene Meinung von der Reinigungskraft des Peroxids ist nach neueren Erkenntnissen nicht mehr haltbar. Proteinablagerungen können vielmehr durch die bleichende Wirkung des Wasserstoffperoxids optisch schlechter erkannt werden, wodurch die Gefahr von Unverträglichkeitsreaktionen eher vergrößert wird. Wasserstoffperoxidlösungen haben zudem den Nachteil, Augenreizungen hervorzurufen, wenn sie nicht ausreichend neutralisiert werden. Zudem kann die verwendete Neutralisationslösung aufgrund des Gehaltes an Konservierungsmittel oder Enzymen Allergien hervorrufen.

Die Tatsache, daß Wasserstoffperoxid in der verwendeten Konzentration nur eine ungenügende Reinigungswirkung aufweist, zeigt auch die Einführung eines Enzymreinigers in Tablettenform, welchem Peroxidlösungen zugesetzt werden können. Der Nachteil dieser Lösung ist die nach wie vor gegebene Reizwirkung des Peroxids sowie die Allergisierungsgefahr durch die Neutralisationslösung. Zudem sind für die Pflege hier drei Komponenten notwendig, nämlich die Wasserstoffperoxidlösung, die Enzymtablette sowie die Neutralisationslösung, was der Patientencompliance eher abträglich ist.

Für die Desinfektion von Kontaktlinsen ist in der US-PS 3 876 768 auch die Verwendung von chlorabspaltenden Verbindungen vorgeschlagen worden. Während die Desinfektion mit chlorfreisetzenden Verbindungen gute Ergebnisse erbringt, ist auch hier die Reinigungswirkung allenfalls mit der des Wasserstoffperoxids zu vergleichen. Bei diesem System kann somit auf eine zusätzliche, getrennte Reinigung nicht verzichtet werden.

Während die bislang bekannten Desinfektionssysteme nur eine ungenügende Reinigungswirkung zeigen, weisen auf der anderen Seite die derzeit verwendeten Reinigungsmittel nur eine ungenügende Desinfektionswirkung auf. Insbesondere die Reinigungsmittel mit oberflächenaktiven Verbindungen können aufgrund der Handhabung (Reiben der Linse mit dem Reiniger zwischen den Fingern) sowie der kurzen Kontaktzeit mit der Linse keine ausreichende Desinfektion gewährleisten. Die zur Reinigung zudem verwendeten Enzymreiniger weisen zwar eine ausgezeichnete Reinigungswirkung auf, wirken aber nur in Verbindung mit dem Konservierungsmittel in der Auflösungsflüssigkeit (z.B. konservierte Kochsalzlösung) desinfizierend. Auf die Nachteile der Verwendung von Konservierungsmitteln wurde bereits eingangs verwiesen.

Aufgabe der Erfindung war daher die Entwicklung eines Pflegesystems, welches eine gute Reinigungswirkung mit einer sicheren Desinfektion verbindet.

Diese Aufgabe wird wie aus dem Anspruch 1 ersichtlich gelöst. Bevorzugte Ausführungsformen des Kontaktlinsenpflegemittels mit desinfizierender Wirkung sind in den Ansprüchen 2 bis 22 beschrieben.

Um die Anwendungssicherheit des Systems zu erhöhen, ist es wünschenswert, daß die Desinfektion und Reinigung optimal abläuft. Die oben erwähnten Pobleme können mit dem erfindungsgemäßen Pflegemittel gelöst werden, wobei eine Kombination von chlorabspaltenden Verbindungen mit Proteasen, Glykosidasen und Lipasen bereitgestellt wird.

Eine solche Kombination war aus dem Stand der Technik nicht ableitbar. In der US-PS 1 527 010 wird sogar ein Reinigungsmittel für Zahnprotesen beschrieben, welches aus Enzymen und einer sauerstoffabspaltenden Verbindung besteht. Auf Seite 3, Zeile 43 ff. wird hier die Lehre vermittelt, daß Hypochlorit oder chlorabspaltende Verbindungen in Kombination mit Enzymen nicht einsetzbar sind, da durch das Chlor eine schnelle Inaktivierung der Enzyme bei der Auflösung des Mittels auftritt. Auch in Ullmann, Band X, Seite 525, wird die Lehre vermittelt, daß Proteasen durch Aktivchlorverbindungen sehr schnell inaktiviert werden. Auf der anderen Seite ist durch Wallhäuser (Praxis der Sterilisation, 3. Auflage 1984, Thieme-Verlag Stuttgart) das Problem der sogenannten Chlorzehrung bekannt. Im oben zitierten Werk wird auf Seite 519 die Lehre vermittelt, daß Chlor beispielsweise mit Aminosäuren und Proteinen reagiert, wodurch ein großer Teil der Chlorwirkung verlorengeht.

Entgegen diesem bestehenden Vorurteil wurde überraschenderweise nun gefunden, daß die Aktivität der Proteasen und auch Glykosidasen durch die chlorfreisetzenden Verbindungen gegenüber einer Rezeptur ohne chlorabspaltende Stoffe nicht relevant vermindert wird.

Durch das erfindungsgemäße Kontaktlinsenpflegemittel können die oben beschriebenen Nachteile herkömmlicher Reinigungs-und Desinfektionssysteme behoben werden. Das erfindungsgemäße Mittel vereinigt Desinfektion und Reinigung und erleichtert so dem Verbraucher die regelmäßige Pflege seiner Kontaktlinsen. Durch diese Kombination wird die Anzahl der Tätigkeiten, zum Beispiel Reinigen mit einem täglichen Reiniger, Abspülen des Reinigers von der Kontaktlinse, Desinfektion, gegebenenfalls Neutralisation, sowie eine einmal in der Woche durchzuführende enzymatische Intensivreinigung auf das Auflösen zum Beispiel einer Tablette in einem geeigneten Lösungsmittel sowie einem Abspülen nach erfolgter Desinfektion und Reinigung reduziert.

Als geeignete Lösungsmittel dienen unter anderem handelsübliche unkonservierte Kochsalzlösungen zur Pflege von Kontaktlinsen, dest. Wasser sowie Leitungswasser. Die im Leitungswasser enthaltenen Calciumverbindungen können durch einen erfindungsgemäß zugesetzten Chelatbildner inaktiviert werden, so daß keine anorganischen Beläge auf den Kontaktlinsen entstehen. Durch die einfache, auf wenige Schritte reduzierte Handhabung wird die Mitarbeit des Patienten entscheidend erleichtert, womit der Pflegezustand von Kontaktlinsen erheblich verbessert werden kann. Durch die Kombination mit chlorfreisetzenden Verbindungen können die Linsen nach kurzem Abspülen oder Schütteln in frischem Lösungsmittel reizfrei getragen werden. Durch die kurze Abspülprozedur wird die Gefahr einer eigenmächtigen Verkürzung der Neutralisationszeit, zum Beispiel bei Wasserstoffperoxid-Systemen, vermindert und so das Problem der Irritationen am Auge stark reduziert. Durch die Möglichkeit der Verwendung von Leitungswasser zum Auflösen des Mittels können zudem die Pflegekosten für den Verbraucher drastisch gesenkt werden.

Da zudem auf Konservierungsmittel wie Thiomersal oder Chlorhexidindigluconat verzichtet werden kann, wird die Gefahr einer Allergisierung durch diese Komponenten vermindert. Durch die in erfindungsgemäßen Mengen verwendeten chlorfreisetzenden Stoffe ist eine sichere Desinfektion der Kontaktlinsen gewährleistet. Lipidablagerungen werden durch die erfindungsgemäße Zugabe von Lipasen, Mucinablagerungen durch erfindungsgemäße Zugabe von Glycosidase und die vorwiegend auftretenden Proteinablagerungen durch die erfindungsgemäß zugegebene Protease oder Proteasen von den Linsen entfernt. Durch den Abbau dieser Ablagerungen werden auch die Bakterien im Inneren solcher Beläge für eine Desinfektion zugänglich.

Das erfindungsgemäße Mittel kann unter Zuhilfenahme der üblichen Hilfsstoffe in Form eines Pulvers, eines Granulates, einer Tablette oder einer Brausetablette hergestellt werden. Unter Tabletten sind auch Mehrschicht-und Manteltabletten zu verstehen. Das Mittel ist dann vor Anwendung in einem geeigneten Lösungsmittel, z.B. einer für die Kontaktlinsenpflege verwendeten unkonservierten Kochsalzlösung aufzulösen. Von den genannten Darreichungsformen erscheint die Tablette oder Brausetablette als besonders geeignet, da sie einfach dosierbar und platzsparend ist.

Neben den üblicherweise für die Herstellung von derartigen Pflegemitteln verwendeten bekannten Hilfsstoffen, wie organische Säuren, z.B. Adi-

pinsäure oder Weinsäure und $CO_2$ abspaltenden Verbindungen, wie Natriumhydrogencarbonat zur Erzeugung des Brauseeffektes sowie Zerfallsverzögerern, Bindemitteln, Gleitmitteln und Füllmitteln können sinnvoll auch noch andere Stoffe zugesetzt werden, um den pH und die Tonizität der Lösung den Gegebenheiten des Auges anzupassen. Weiterhin können geeignete Enzymaktivatoren sowie Chelatbildner, z.B. Natriumethylendiamintetraessigsäure, zugesetzt werden. Der Zusatz von Chelatbildnern bewirkt zudem auch eine Reinigungswirkung gegen anorganische Ablagerungen, insbesondere Calciumablagerungen, auf Kontaktlinsen. Ein weiterer Vorteil von Chelatbildnern ist die Möglichkeit, auch Leitungswasser zum Auflösen des erfindungsgemäßen Mittels zu verwenden, da die Wasserhärte des Leitungswassers (Calciumverbindungen) durch den Chelatbildner vermindert wird.

Die im erfindungsgemäßen Pflegemittel enthaltenen chlorfreisetzenden Mittel bzw. Verbindungen sind an sich bekannt (siehe Wallhäuser, "Praxis der Sterilisation", 3. Auflage 1984, Thieme-Verlag, Stuttgart, Seite 517 ff.). Bevorzugt sind dabei die organischen Chlorverbindungen wie das Chloramin B - N-Chlorbenzolsulfonamido-Natrium - Benzolsulfochloramin-Na - Neomagnol, Chloramin T - p-Toluolsulfonchloramid-Na - Chlorazene - Aktiven - Chlorazone - Clorina - Euclorina, Dichloramin T - p-Toluolsulfodichloramid, Halazon - p-Dichlorsulfamylbenzoesäure, Halane - Cyanursäurederivate und Trichlormelamin - TCM. Insbesondere bevorzugt sind p-Dichlorsulfamylbenzoesäure, Dichlorisocyanursäure und Trichlorisocyanursäure.

Als die Reinigung vermittelnde Komponente wird erfindungsgemäß eine Protease aus der Reihe der Serin-Histidin-Proteasen vorgeschlagen. Bei diesen Proteasen wird das aktive Zentrum des Enzyms durch die Aminosäuren Serin und Histidin charakterisiert. Bevorzugt ist hierbei Trypsin, Chymotrypsin und Subtilisin A. Die Proteasen können erfindungsgemäß sowohl alleine als auch in Kombination eingesetzt werden. Zur Verbesserung der Wirkung ist es bevorzugt, neben der Protease auch eine Glykosidase einzusetzen. Besonders geeignet erscheint α-Amylase. Als natürliche Kombination von Trypsin, Chymotrypsin, α-Amylase und anderen Enzymen kann erfindungsgemäß auch Pankreatin eingesetzt werden. Zusätzlich kann zur Entfernung von Lipidablagerungen eine Lipase zugesetzt werden. Geeignet ist hier Pankreaslipase sowie mikrobiologische Lipasen, z.B. aus Rhizopusoder Candida-Arten. Da die Mengenanteile der einzelnen Enzyme von ihrer Aktivität abhängen, ist die Aktivität gemessen mit den Methoden der FIP (Fédération Internationale de Pharmaceutique) angegeben. Der Fachmann kann aus den FIP-Einhei

ten pro Gramm Enzym die benötigte Menge Enzym pro Zubereitung mittels des Dreisatzes berechnen. Bei Aktivitätsverlusten während der Herstellung muß ein entsprechender Zuschlag vorgenommen werden. Die Menge an verfügbarem Chlor in der Pflegemittellösung soll 0,5-100 ppm, vorzugsweise 0,5-20 ppm betragen. Die proteolytische Aktivität soll 1-1000 E, insbesondere 5-120 E, vorzugsweise 5-60 E (nach FIP bestimmt) ausmachen. Die glycolytische Aktivität soll 1-1000 E, insbesondere 1-60 E, vorzugsweise 1-30 E (nach FIP bestimmt) ausmachen. Die lipolytische Aktivität soll 1-1000 E, insbesondere 1-120 E, vorzugsweise 1-20 E (nach FIP bestimmt) betragen.

Das erfindungsgemäße Pflegemittel kann als Pulver, z.B. in Form von Briefchen, als Tablette, Granulat und als Brausetablette, in den Handel gelangen. Hierbei ist zwischen den einfachen Gemischen der desinfizierenden und reinigenden Komponenten und Applikationsformen mit aufeinanderfolgendem Wirkungseintritt der chlorfreisetzenden Verbindung und der Enzyme zu unterscheiden. Die letzteren, der Einfachheit halber 2-Phasen-Applikationsformen genannten Zubereitungen, lassen sich in Form von Mehrschicht-oder Manteltabletten herstellen. 2-Phasen bedeutet in diesem Zusammenhang eine zeitliche Verzögerung des Eintritts der Enzymwirkung, gegenüber dem Eintritt der desinfizierenden Wirkung der chlorfreisetzenden Verbindung. Bei Mehrschicht-Tabletten ist eine Schicht der Tablette als Desinfektionsschicht, eine weitere Schicht als Reinigungsschicht konzipiert. Zur Verbesserung der Lagerungsstabilität können beide Schichten auch durch neutrale Zwischenschichten getrennt werden. Die die chlorfreisetzende Verbindung enthaltende Desinfektionsschicht löst sich schnell auf und tötet die auf den Belägen der Kontaktlinse und der Kontaktlinse selbst eventuell vorhandenen Bakterien.

Diese Schicht kann vorzugsweise als Brauseschicht aufgebaut sein.

Während der Desinfektion setzt die langsame Lösung der zweiten Schicht, der Reinigungsmittelschicht, ein. Dabei wird von den Enzymen das sich in der Lösung befindliche Chlor sowie eventuell noch in den chlorabgebenden Verbindungen befindliche Chlor langsam verbraucht und somit desaktiviert, ohne daß es überraschenderweise zum Abbau bzw. der Desaktivierung der Enzyme selbst kommt. Nach der Desinfektion setzt also eine problemlose Reinigung der Kontaktlinsen ein, gleichzeitig verbunden mit einer Abnahme der Wirkung des Chlors. Nach Beendigung der Reinigung ist nur noch eine geringe Menge der chlorfreisetzenden Verbindung vorhanden, was für die Reizfreiheit der Kontaktlinsen vorteilhaft ist. Die Bereitstellung einer 2-Phasen-Applikationsform hat auch den Vorteil, daß zu Beginn die Desinfektion so eingestellt werden kann, daß sie im sauren Bereich erfolgt. Anschließend wird die Formulierung der nächsten Aktivschicht, d.h. der Reinigungsschicht, so eingestellt, daß sich der pH der Anwendungslösung vom sauren zum alkalischen Bereich verschiebt und damit die gewünschte Reinigung im pH-Optimum der Enzyme erfolgen kann. Hierzu können in den einzelnen Schichten die notwendigen üblichen Hilfsstoffe eingesetzt werden. Die Einwirkzeit der 2-Phasen-Applikationsformen richtet sich nach dem Gehalt der chlorfreisetzenden Verbindung und der Enzymaktivität und kann bis zu 12 Stunden betragen.

Die 2-Phasen-Applikationsform kann auch als sogenannte Manteltablette hergestellt werden, wobei sich die chlorfreisetzende Verbindung in der schnell auflösenden äußeren Mantelschicht befindet. Die erfindungsgemäß zugesetzten Enzyme befinden sich im Tablettenkern, welcher sich langsam auflöst. Gegebenenfalls kann der Kern durch geeignete Polymerüberzüge in der Lösungsgeschwindigkeit noch vermindert werden. Geeignete Überzüge sind aus der Pharmazie bekannt und dienen dort zum Retardieren von Arzneistoff-Freisetzungen. Geeignet sind z.B. Celluloseacetatphthalat, Methacrylsäurederivate, Hydroxypropylmethylcellulose und andere. Der zeitliche Verlauf der Auflösung der einzelnen Schichten bzw. von Mantel und Kern hängt von der Menge an chlorfreisetzender Verbindung und der Enzymmenge ab.

Die Auflösungsgeschwindigkeit muß, wie dem Fachmann ohne weiteres geläufig ist, so aufeinander abgestimmt werden, daß eine optimale Desinfektion und eine optimale Reinigung gewährleistet ist.

Wie bereits erwähnt, kann die 2-Phasen-Applikationsform als Mehrschichttablette oder Manteltablette hergestellt werden. Die Herstellung von Mehrschichttabletten oder Manteltabletten ist dem Fachmann geläufig und wird bei Reinigungsmitteln für Zahnprothesen und in der Pharmazie zur Erzielung von Retardeffekten bereits angewandt. Im folgenden wird die Herstellung von 2-Schichttabletten beschrieben. Andere galenische Zubereitungen mit dem gleichen Effekt, nämlich einer zeitlichen Verzögerung des Wirkungseintritts der Enzyme gegenüber dem Wirkungseintritt der chlorabspaltenden Verbindung, können auch verwendet werden.

Ebenso kann für die Darstellung des erfindungsgemäßen Mittels auch die Herstellung von Pulvern, Granulaten, Tabletten und Brausetabletten ohne 2-Phaseneffekt geeignet sein. Trotz der bekannten Chlorzehrung, d.h. der Bindung von freiem Chlor an organische Substanzen, z.B. Enzyme, bleibt die desinfizierende Wirkung des Chlors überraschenderweise erhalten. Durch die Anlagerung des Chlors an die Enzyme geht nur eine Teilmenge verloren, so daß der Rest für die Desinfektion zur Verfügung steht. Diese Zubereitungen benötigen zwar eine längere Zeit für die Desinfektion, sind jedoch einfacher herzustellen wie die 2-Phasen-Applikationsformen.

Auf die ausführliche Erläuterung zur Herstellung dieser einfacheren Darreichungsformen wird verzichtet, da sie als dem Fachmann bekannt vorausgesetzt werden kann.

Die Herstellung von 2-Schichttabletten erfolgt auf speziellen Tablettenmaschinen, bei welchen zunächst eine Schicht durch Verpressen in der Matrize vorverdichtet wird. Im Anschluß an diese erste Pressung erfolgt dann die Beschickung der Matrize mit dem Füllgut für die zweite Schicht und anschließend die Komprimierung, in welcher beide Schichten verdichtet werden.

Beispiel 1

Trypsin (3000 E/g nach FIP)          15 mg
α-Amylase (10000 E/g nach FIP)       5 mg
Lactose, wasserfrei          25 mg
Polyethylenglykol 6000          5mg

werden in geeigneten Vorrichtungen homogen gemischt und im ersten Schritt der Tablettierung vorverdichtet.

Adipinsäure, wasserfrei          28 mg
Natriumhydrogencarbonat          34 mg
p-Dichlorsulfamylbenzoesäure          0,16 mg

werden mit geeigneten Methoden homogen vermischt und unter Verwendung von geeigneten Bindemitteln, z.b. Polyvinylpyrrolidon, Polyethylenglykol 6000, Hydroxypropylmethylcellulose wasserfrei granuliert. Als Lösungsmittel für die Bindemittel dienen z.B. Methylenchlorid, Chloroform, Isopropanol oder Ethanol mit geringem Wasseranteil. Das Granulat wird in der zweiten Stufe der Tablettierung zusammen mit der vorverdichteten ersten Schicht zu 2-Schichttabletten verpreßt. Beim Lösen dieser Tablette in 10 ml destilliertem Wasser ergibt sich ein Gehalt von 8 ppm verfügbarem Chlor. Nach kompletter Auflösung der zweiten Schicht beträgt die proteolytische Aktivität 45 E/10 ml, bestimmt nach FIP. Die glycolytische Aktivität liegt bei 50 E/10 ml nach FIP.

Beispiel 2

Trypsin (1300 E/g nach FIP)          10 mg
Chymotrypsin (2500 E/g nach FIP)          10 mg
α-Amylase (10000 E/g nach FIP)          10 mg
Polyvinylpyrrolidon (Kollidon [R]K 30)          5 mg
Sorbit          30 mg

werden in geeigneten Vorrichtungen homogen gemischt und im ersten Schritt der Tablettierung vorverdichtet.

　　Weinsäure　25 mg
　　Natriumhydrogencarbonat　50 mg
　　Dichlorisocyanursäure　0,07 mg

werden mit geeigneten Methoden homogen vermischt und unter Verwendung von geeigneten Bindemitteln, z.B. Polyvinylpyrrolidon, Polyethylenglykol 6000 oder Hydroxypropylmethylcellulose wasserfrei granuliert. Als Lösungsmittel dienen z.B. Methylenchlorid, Chloroform, Isopropanol oder Ethanol mit möglichst geringem Wassergehalt. Das Granulat wird dann in der zweiten Stufe der Tablettierung zusammen mit der vorverdichteten Erstschicht zu 2-Schichttabletten verpreßt.

Beim Lösen dieser Tablette in 10 ml destilliertem Wasser ergibt sich ein Gehalt von 5 ppm verfügbarem Chlor. Die proteolytische Gesamtaktivität nach Auflösen der zweiten Schicht liegt bei 38 E/10 ml, bestimmt nach FIP. Die glycolytische Aktivität nach Auflösen der Enzymschicht beträgt 100 E/10 ml, bestimmt nach FIP.

## Beispiel 3

　　Pankreatin (proteolytische Aktivität 3000 E/g nach FIP, Amylaseaktivität 7500 E/g nach FIP, lipolytische Aktivität 7500 E/g nach FIP)　15 mg
　　Mannit　30 mg
　　Polyethylenglykol 6000　10 mg

werden in geeigneten Vorrichtungen homogen vermischt und im ersten Schritt der Tablettierung vorverdichtet.

　　Adipinsäure, wasserfrei　28 mg
　　Natriumhydrogencarbonat　64 mg
　　Natriumethylendiamintetraessiosäure　20 mg
　　Dichlorisocyanursäure　0,11 mg

werden mit geeigneten Methoden homogen vermischt und unter Verwendung von geeigneten Bindemitteln, z.B. Polyvinylpyrrolidon, Polyethylenglykol 6000 oder Hydroxypropylmethylcellulose wasserfrei granuliert. Als Lösungsmittel dienen z.B. Methylenchlorid, Chloroform, Isopropanol und Ethanol mit möglichst geringem Wassergehalt. Beim Auflösen der Tablette in 7,5 ml destilliertem Wasser ergibt sich ein Gehalt von 9,5 ppm verfügbarem Chlor, sowie nach Auflösen der zweiten Schicht eine proteolytische Aktivität von 45 E/7,5 ml, bestimmt nach FIP, eine glycolytische Aktivität von 112,5 E/7,5 ml, bestimmt nach FIP, sowie eine lipolytische Aktivität von 112,5 E/7,5 ml, bestimmt nach FIP.

## Beispiel 4

Es wird eine einfache Tablette folgender Zusammensetzung hergestellt:

　　Subtilisan A (7000 E/g nach FIP)　5 mg
　　α-Amylase (5000 E/g nach FIP)　5 mg
　　Rhizopus-Lipase (7000 E/g nach FIP)　3 mg
　　Adipinsäure, wasserfrei　28 mg
　　NaHCO$_3$　64 mg
　　Na-EDTA　20 mg
　　p-Dichlorsulfamylbenzoesäure　0,30 mg
　　Polyethylenglykol 6000　5 mg
　　Polyvinylpyrrolidon (Kollidon$^R$K 30)　5 mg
　　NaCl　30 mg

Beim Lösen der Tablette in 10 ml Leitungswasser ergibt sich ein Gehalt von 15 ppm verfügbarem Chlor. Die proteolytische Aktivität beträgt 35 E/10 ml (FIP), die glycolytische Aktivität liegt bei 25 E/10 ml (FIP), die lipolytische Aktivität beträgt 21 E/10 ml (FIP).

## Ansprüche

1. Kontaktlinsenpflegemittel mit desinfizierender Wirkung mit einem Gehalt an chlorfreisetzenden Verbindungen, **dadurch gekennzeichnet,** daß es zur Kombination mit einer reinigenden Wirkung

　　a. eine chlorfreisetzende Verbindung enthält und eine Konzentration von 0,5 bis 100 ppm verfügbarem Chlor in der Gebrauchslösung aufweist,

　　b. eine oder mehrere Proteasen enthält und eine proteolytische Aktivität von 1 bis 1000 E (FIP-Einheiten) aufweist,

　　c. übliche Formulierungshilfsstoffe enthält.

2. Kontaktlinsenpflegemittel nach Anspruch 1, **dadurch gekennzeichnet,** daß es eine chlorfreisetzende Verbindung enthält und die Konzentration an verfügbarem Chlor in der Gebrauchslösung 0,5 bis 20 ppm beträgt.

3. Kontaktlinsenpflegemittel nach einem der Ansprüche 1 oder 2, **dadurch gekennzeichnet,** daß es eine oder mehrere Proteasen enthält und eine proteolytische Aktivität von 5 bis 120 E (FIP-Einheiten) aufweist.

4. Kontaktlinsenpflegemittel nach Anspruch 3, **dadurch gekennzeichnet,** daß es eine oder mehrere Proteasen enthält und eine proteolytische Aktivität von 5 bis 60 E (FIP-Einheiten) aufweist.

5. Kontaktlinsenpflegemittel nach einem der vorhergehenden Ansprüche 1 bis 4, **dadurch gekennzeichnet,** daß es als chlorfreisetzende Verbindung p-Dichlorsulfamylbenzoesäure, Dichlorisocyanursäure und/oder Trichlorisocyanursäure enthält.

6. Kontaktlinsenpflegemittel nach einem der Ansprüche 1 bis 5, **dadurch gekennzeichnet, daß** es als Protease oder Proteasen Enzyme vom Serin-Histidin-Typ enthält.

7. Kontaktlinsenpflegemittel nach Anspruch 6, **dadurch gekennzeichnet, daß** es als Proteasen Trypsin, Chymotrypsin und/oder Subtilisin A alleine oder in Kombination enthält.

8. Kontaktlinsenpflegemittel nach einem der Ansprüche 1 bis 7, **dadurch gekennzeichnet, daß** es Pankreatin enthält.

9. Kontaktlinsenpflegemittel nach einem der Ansprüche 1 bis 8, **dadurch gekennzeichnet, daß** es zusätzlich eine Glycosidase enthält und eine glycolytische Aktivität von 1 bis 1000 E (FIP-Einheiten) aufweist.

10. Kontaktlinsenpflegemittel nach Anspruch 9, **dadurch gekennzeichnet, daß** es eine Glycosidase enthält und eine glycolytische Aktivität von 1 bis 60 E (FIP-Einheiten) aufweist.

11. Kontaktlinsenpflegemittel nach Anspruch 10, **dadurch gekennzeichnet, daß** es eine Glycosidase enthält und eine glycolytische Aktivität von 1 bis 30 E (FIP-Einheiten) aufweist.

12. Kontaktlinsenpflegemittel nach einem der Ansprüche 9 bis 11, **dadurch gekennzeichnet, daß** es als Glycosidase $\alpha$-Amylase enthält.

13. Kontaktlinsenpflegemittel nach einem der Ansprüche 1 bis 12, **dadurch gekennzeichnet, daß** es zusätzlich eine Lipase enthält und eine Lipase-Aktivität von 1 bis 1000 E (FIP-Einheiten) aufweist.

14. Kontaktlinsenpflegemittel nach Anspruch 13, **dadurch gekennzeichnet, daß** die Lipase-Aktivität 1 bis 120 E (FIP-Einheiten) beträgt.

15. Kontaktlinsenpflegemittel nach Anspruch 13, **dadurch gekennzeichnet, daß** die Lipase-Aktivität 1 bis 20 E (FIP-Einheiten) beträgt.

16. Kontaktlinsenpflegemittel nach Anspruch 13 bis 15, **dadurch gekennzeichnet, daß** es als Lipase Pankreaslipase enthält.

17. Kontaktlinsenpflegemittel nach Anspruch 13 bis 15, **dadurch gekennzeichnet, daß** es als Lipase eine mikrobiologische Lipase aus Rhizopus- oder Candida-Arten enthält.

18. Kontaktlinsenpflegemittel nach einem der Ansprüche 1 bis 17, **dadurch gekennzeichnet, daß** es einen Chelatbildner enthält.

19. Kontaktlinsenoflegemittel nach Anspruch 18, **dadurch gekennzeichnet, daß** es EDTA enthält.

20. Kontaktlinsenpflegemittel nach einem der Ansprüche 1 bis 19, **dadurch gekennzeichnet, daß** es zusätzlich einen oder mehrere Enzymaktivatoren enthält.

21. Verfahren zum Pflegen von Kontaktlinsen durch Behandlung mit chlorfreisetzenden Verbindungen, **dadurch gekennzeichnet, daß** man entsprechend einem der Ansprüche 1 bis 20 den Wirkungseintritt der Enzyme gegenüber dem Wirkungseintritt der chlorfreisetzenden Verbindung zeitlich verzögert.

22. Verfahren nach Anspruch 21, **dadurch gekennzeichnet, daß** man den pH der Gebrauchslösung während der Auflösung des erfindungsgemäßen Mittels vom sauren in den alkalischen Bereich verschiebt.